# EUROPEAN PATENT SPECIFICATION

(11) **EP 1 299 063 B1**
(45) Date of publication and mention of the grant of the patent: **29.03.2006**
(21) Application number: 01950154.3
(22) Date of filing: 09.07.2001
(51) Int. Cl.: A61F 13/56

(54) **ABSORBENT ARTICLE WITH FASTENING AREAS OF DIFFERENT ATTACHABILITIES**
ABSORBIERENDER ARTIKEL MIT VERSCHLUSSZONEN UNTERSCHIEDLICHER EIGENSCHAFTEN
ARTICLE ABSORBANT A ZONES DE FIXATION A DIFFERENTES POSSIBILITES D'ATTACHE

(30) Priority: 11.07.2000 SE 0002636
(43) Date of publication of application: 09.04.2003
(73) Proprietor: SCA Hygiene Products AB, 405 03 Göteborg (SE)
(72) Inventor: RÖNNBERG, Peter, S-431 33 Mölndal (SE); SANDIN, C cile, S-431 36 Mölndal (SE); FERNFORS, Ingemar, S-431 38 Mölndal (SE)
(74) Representative: Romare, Laila Anette
(86) International application number: PCT/SE2001/001584
(87) International publication number: WO 2002/003901

(56) References cited:
- EP-A1- 0 719 534
- EP-A2- 0 893 115
- WO-A1-00/37016
- WO-A1-98/18422
- US-A- 5 423 789
- US-A- 5 605 735

## Description

### TECHNICAL FIELD

The present invention relates to a product, which product is absorbent or can act as a support for an absorbent product, with the product having a longitudinal direction, a transverse direction, an upper side and a lower side, and the product comprises a front waist section, a back waist section and a middle section which is located between the waist sections, which front waist section is provided with front fastening members and has first regions in connection with the said front fastening members, which back waist section is provided with back fastening members and has second regions in connection with said back fastening members, with the said front or back fastening members being able to receive and co-operate with the other of the said front or back fastening members, respectively, and a first fastening ability between the said front and back fastening members thereby being exhibited, and the said first fastening ability comprises a first shearing strength and a first peeling strength.

### TECHNICAL BACKGROUND

Products, which products are absorbent or can act as supports for absorbent articles which, during use, are fastened around a user's body at, for example, the waist or the hips, can, for this purpose, be provided with fastening members. The fastening members can, for example, be re-sealable tapes having an appurtenant reception zone (TLZ) consisting of reinforcing material, or a combination of hook and loop members (e.g. Velcro®). Since the cost of the fastening members, in particular the cost of the combination of hook and loop members, has constituted a considerable part of the total cost of the said products, there has been a desire to reduce the cost of the fastening members. One way of reducing this cost is to make the fastening members as small as possible. Since technical development of fastening members has resulted in a decrease in the fastening area, i.e. the contact area through which the fastening members receive and co-operate, which is required for obtaining sufficient shearing or peeling strength, it is also possible to make the fastening members small. In the present context, shearing strength denotes the ability, per unit of area, of the co-operating fastening members to withstand shearing forces, which act parallel to the said fastening area, and, in the present context, peeling strength denotes the ability, per unit of length, of the co-operating fastening members to withstand peeling forces, which act perpendicular to the said fastening area. Fastening ability is the expression that is hereinafter employed to designate shearing strength and peeling strength taken together. The fastening area which is required for sufficient fastening ability is now so small that sufficient fastening ability can be exhibited even if the fastening members only receive each other partially. This simplifies the fastening of the product due to the fact that the relatively small fastening members do not need to receive each other fully for the function of the product to be acceptable. Furthermore, the fact that the fastening members receive each other partially also provides certain possibilities for adjusting the fit of the product. However, the fact that the fastening members receive each other partially has also meant that non-activated (inactive) parts of the fastening members are exposed to their environment and consequently run the risk of catching in objects in the environment, for example the user's underclothes, bedclothes or the like, a situation which in turn increases the risk of the product being opened unintentionally.

In the case of a product in accordance with the present invention, there is no longer any risk of the parts of the fastening members which have not been received catching in objects in the environment and consequently there is no risk, either, of the product being opened unintentionally.

### DISCLOSURE OF THE INVENTION

The present invention relates to a product, which product is absorbent or can act as a support for an absorbent article, with the product having a longitudinal direction, a transverse direction, an upper side and a lower side. Furthermore, the product comprises a front waist section, a back waist section and a middle section which is located between the waist sections, with the said front waist section being provided with front fastening members and having first regions in connection with the said front fastening members, and the said back waist section being provided with back fastening members and having second regions in connection with the said back fastening members. The said front or back fastening members can receive and co-operate with the other of the said front or back fastening members, respectively, and a first fastening ability between the said front and back fastening members is thereby exhibited, with the said first fastening ability comprising a first shearing strength and a first peeling strength. Furthermore, the said front fastening members or the said back fastening members have a first extent principally in the longitudinal direction of the product and the other of the said front fastening members or said back fastening members, respectively, have a second extent principally in the longitudinal direction of the product. An extent ratio, which extent ratio is at least 1.5, is exhibited between the said second extent and the said first extent. Furthermore, the said first regions can receive, and co-operate with, the said back fastening members, and a second fastening ability is thereby exhibited between the said first regions and the said back fastening members, and/or the said second regions can receive, and co-operate with, the said front fastening members, and a third fastening ability is thereby exhibited between the said second regions and the said front fastening members. The said second fastening ability comprises a second shearing strength and a second peeling strength, and the said third fastening ability comprises a third shearing strength and a third peeling strength. Furthermore, a first shearing strength ratio is exhibited between the said second shearing strength and the said first shearing strength, and a second shearing strength ratio is exhibited between the said third shearing strength and the said first shearing strength, both of which shearing strength ratios are less than 1.0, and a first peeling strength ratio is exhibited between the said second peeling strength and the said first peeling strength, and a second peeling strength ratio is exhibited between the said third peeling strength and the said first peeling strength, both of which peeling strength ratios are less than 10, with at least one of the said second and third shearing strengths being greater than zero and/or at least one of the said second and third peeling strengths being greater than zero.

The said product can be an absorbent disposable product of the type which is fastened around a user's body at, for example, the waist or the hips, for example napkins for babies or individuals who suffer from incontinence, or an absorbent or non-absorbent product of the underpants type having resealing devices on a level with the user's waist or hips. Furthermore, the said product can be an absorbent or non-absorbent product of a type in which back or front fastening members comprise a belt having fastening devices, with it being possible for the said belt to be integrated with, or separate from, the said product: see the products which are described in EP, A, 0696911. When the said product is a non-absorbent product, the said product can act as a support for an absorbent article, for example a napkin, a sanitary towel, a panty shield or an incontinence protection.

Furthermore, the longitudinal direction of the product extends from the back waist section to the front waist section, and the transverse direction of the product goes transversely to the said longitudinal direction, with the back waist section being the waist section which bears against a user's seat or back and the front waist section being the waist section which bears against a user's abdomen when using the product. The middle section which is located between the waist sections unites the two waist sections. Furthermore, when the product is being used, the upper side of the product is the side which is facing a user and its lower side is the side which is facing away from a user.

It is to be noted that the division of the said product into a back waist section, a front waist section and a middle section is not to be understood as meaning that there are boundaries between the different sections, but is first and foremost intended to facilitate the description of the said product, using as a starting point the differences which exist between the different sections depending on how they are intended to be placed in relation to a user's body. Accordingly, the transition between the different sections does not take place at any definite transverse lines but rather within diffuse transitional regions.

The said front fastening members or said back fastening members have a first extent principally in the longitudinal direction of the product, and the other of the said front fastening members or said back fastening members, respectively, have a second extent principally in the longitudinal direction of the product, with an extent ratio, which extent ratio is at least 1.5, being exhibited between the said second extent and the said first extent. Furthermore, the front and back fastening members of the product can both consist of one or more fastening members each, with it being possible for one of the said front and back fastening members to be a continuous fastening member, or it being possible for one or both of the said front or back fastening members to consist of several smaller fastening members. Furthermore, the said fastening members can have a suitable shape which is optional, for example a square, rectangular, circular, oval or tapering shape. The said back or front fastening members can also comprise a belt having appurtenant fastening devices, with it being possible for the said belt to be integrated with, or separate from, the said product, see the already mentioned EP, A, 0696911. Furthermore, the said front and back fastening members can have their extents substantially transverse to each other. The front and back fastening members of the product can, for example, be of the type consisting of re-sealable tapes having appurtenant reception zones (TLZ) consisting of reinforcing material, combinations of hook and loop members (for example Velcro®), hybrid variants, for example STEMWEB® from 3M, fastening members which are described in GB, A, 2303821, combinations of adhesive fastening members and hook/loop members, or be of another suitable type which is known to a person skilled in the art.

The fastening member which is described in GB, A, 2303821 is a fastening member in a product in accordance with the present invention, in which the fastening member comprises a pressure-sensitive adhesive layer and an essentially incompressible, non-adhesive covering layer having a thickness which does not exceed 0.5 mm and exhibiting a multiplicity of through apertures or pores, which covering layer is applied over, and affixed to, the surface of the adhesive layer which is facing away from the product. The reciprocal distance between two adjacent apertures or pores in the covering layer does not exceed 3 mm. The said fastening member can be fastened to porous textile or textile-like surfaces but exhibits a low ability, or no ability, to adhere to smooth materials having low drapability or to itself, with the said pressure-sensitive adhesive layer consisting, for example, of hot-melt-type adhesives, other types of adhesives having suitable properties, such as water-based adhesives, hardening adhesives or adhesives containing organic solvents, or a double-sided tape, and the said covering layer consisting, for example, of a plastic net, a perforated plastic film or a layer of non-woven material or of a sparse woven fabric.

Furthermore, the said first regions in connection with the said front fastening members, and/or the said second regions, in connection with the said back fastening members, consist of a material which has a character, for example surface structure, adhesion ability or the like, for example non-woven materials, hot-melt-type adhesives, fibre cloths, textile materials, loop and hook materials or films, which make possible reception of, and co-operation with, the fastening members to which the regions are not connected.

The term non-woven material refers to non-woven fabrics. Suitable non-woven materials can consist of natural fibres, such as cellulose or cotton, or of synthetic fibres, such as polyethene, polypropene, polyester, polyurethane, nylon or regenerated cellulose. It is also possible to use non-woven material which is produced from fibres which include two or more components and from mixtures of different fibre types.

The said first, second and third fastening abilities are terms for the ability of the co-operating parts to withstand, per unit of area (shearing strength) and per unit of length (peeling strength), the separating forces which act on the co-operating parts when the said product is being used and is attached to a user. The co-operating parts are the front and back fastening members, the front fastening member and the said second region, and the back fastening member and the said first region, respectively. The said separating forces arise from forces which act on the co-operating parts and which tend to cause the co-operating parts to come apart, to loosen or to be moved. Furthermore, the said separating forces comprise both shearing forces and peeling forces. The term shearing forces comprises the propagated forces which act substantially tangentially to the extent of the co-operating parts and can be assumed to be parallel to the said extent. The term peeling forces comprises the propagated forces which act substantially longitudinally to the extent of the co-operating parts and can be assumed to be perpendicular to the said extent. The said first, second and third fastening abilities all consist of shearing strength (the said first, second and third shearing strength, respectively) and peeling strength (the said first, second and third peeling strength, respectively), with the said shearing strength constituting the ability of the co-operating parts to withstand, per unit of area, the said shearing forces, and the said peeling strength constituting the ability of the co-operating parts to withstand, per unit of length, the said peeling forces.

The said shearing strength can be measured by means of a pulling test in which the co-operating parts are pulled in opposite directions and substantially parallel to the extent of the co-operating parts. A method of measuring the ability to withstand shearing forces is described in detail in the patent US, A, 4699622, Toussant et al., and this patent is included herewith by reference in order to describe the method of measuring the shearing strength, i.e. the ability, per unit of area, of co-operating parts to withstand shearing forces.

The said peeling strength can be measured by means of a pulling test in which one of the co-operating parts is pulled from the other co-operating part at an angle which is approximately 135° with respect to the extent of the co-operating parts. A method of measuring the ability to withstand peeling forces is described in detail in the patent US, A, 4846815, Scripps, and this patent is included herewith by reference for the purpose of describing the method of measuring the peeling strength, i.e. the ability, per unit of length, of co-operating parts to withstand peeling forces.

The said first shearing strength and the said first peeling strength, which are exhibited when the said front and back fastening members co-operate, the said second shearing strength and the said second peeling strength, which are exhibited when the said first regions co-operate with the said back fastening members, and also the said third shearing strength and the said third peeling strength, which are exhibited when the said second regions co-operate with the said front fastening members, can all be measured using the methods which have been referred to above.

By using the said methods for measuring shearing strength and peeling strength, i.e. the fastening ability, it has been found that a product according to the present invention exhibits a first shearing strength ratio between the said second shearing strength and the said first shearing strength and also a second shearing strength ratio between the said third shearing strength and the said first shearing strength, both of which shearing strength ratios are less than 1.0, and a first peeling strength ratio between the said second peeling strength and the said first peeling strength and also a second peeling strength ratio between the said third peeling strength and the said first peeling strength, both of which peeling strength ratios are less than 10, with at least one of the said second and third shearing strengths being greater than zero and/or at least one of the said second and third peeling strengths being greater than zero.

The present invention provides further products in which the said extent ratio between the said second extent and the said first extent is at least 2, 3, 4 or 5.

The present invention furthermore provides products in which the said extent ratio between the said second extent and the said first extent is at least 10 or 15.

The present invention provides still further products in which the said extent ratio between the said second extent and the said first extent is at least 25, 30, 35, 40, 45 or 50.

In further embodiments, the present invention provides products in which the said shearing strength ratios are less than 0.8, 0.6 or 0.4.

In yet further embodiments, the present invention provides products in which the said peeling strength ratios are less than 7, 4 or 2.

In yet another embodiment, the present invention provides a product in which the said first shearing strength ratios and/or the said second shearing strength ratios are greater than 0.01, and/or the said first peeling strength ratios and/or the said second peeling strength ratios are greater than 0.05.

Further embodiments according to the present invention provide products in which the said first shearing strength ratios and/or the said second shearing strength ratios are greater than 0.05, 0.1 or 0.2, and/or the said first peeling strength ratios and/or the said second peeling strength ratios are greater than 0.1, 0.3 or 0.5.

Yet another embodiment provides a product in accordance with the present invention in which the said first regions and the said second regions, which make possible the reception of fastening members and co-operation with these members, can be of an optional size; for example, the said first regions or the said second regions can constitute the whole of the said upper side or the whole of the said lower side, or a relatively large area of the said front waist section or the said back waist section, and/or be substantially restricted to a relatively small area. The said first regions and second regions can both consist of one, two or more part regions, and the said first regions and second regions can be homogeneous or heterogeneous.

Yet another embodiment provides a product in accordance with the present invention which comprises a first fastening area, i.e. the contact area through which the said front and back fastening members receive and co-operate, a second fastening area, i.e. the contact area through which the said first regions co-operate with the said back fastening members, and also a third fastening area, i.e. the contact area through which the said second regions co-operate with the said front fastening members. The said first, second and/or third fastening areas can be divided up into several areas which are separate from each other. The sum of the said first fastening area is less than or equal to the sum of the said second fastening area, and the sum of the said first fastening area is less than or equal to the sum of the said third fastening area.

In a product in accordance with the invention, the said front and/or back fastening member is/are arranged on projecting tongues, which projecting tongues are arranged at the said front and/or back waist section. The said projecting tongues principally extend in the transverse direction of the product.

In one embodiment, the invention provides a product in which the said first regions and/or the said second regions consist of non-woven material.

In yet another embodiment, the invention provides a product in which the lower side of a product consists of a non-woven material, which non-woven material is laminated to a liquid-impermeable layer.

One embodiment according to the invention provides a product in which the said fastening members consist of co-operating hook and loop elements.

Another embodiment according to the invention provides a product in which the said fastening members consist of co-operating, re-sealable tapes having appurtenant reception zones consisting of reinforcing material.

Yet another embodiment of the invention provides a product, which product is an absorbent product which comprises an absorbent body, with it being possible for the absorbent body to contain suitable materials which are known to a person skilled in the art, for example natural materials, for example cellulose fibres (for example in fluffed form), cotton fibres, peat, or similar, synthetic materials, for example absorbent or non-absorbent synthetic fibres, super-absorbent materials, i.e. polymers having the ability to absorb several times their own weight of a fluid, or suitable mixes thereof. Furthermore, the absorbent body can also contain other components such as shape-stabilising members, liquid-spreading members, binding agents such as thermoplastic fibres, or absorbent foam material.

Yet another embodiment of the invention provides a product, which product is a non-absorbent product.

A product according to the present invention allows the front fastening members and the back fastening members to be located such that the front fastening members and the back fastening members only partially receive each other when the product is fastened, without the parts of the fastening members which have not been received being exposed to the environment of the product.

Furthermore, a product according to the present invention allows the front fastening members and the back fastening members to be located such that the front fastening members and the back fastening members only partially receive each other without there being any risk of those parts of the fastening members which have not been received catching in objects in the environment of the product, for example the user's underclothes, bedclothes or the like. The present invention thereby provides a product in which the costs of fastening members can be kept low at the same time as fastening of the product is simplified and there are certain possibilities of adjusting the fit of the product, and in which there is no risk of the product opening unintentionally.

### DESCRIPTION OF THE FIGURES

Exemplary embodiments, which describe the invention but which in no way limit the invention, are described below.
- Figure 1: is a view of a product which does not form part of the present invention seen from the front.
- Figure 2: is a view of yet another product according to the present invention, which product is seen from above and is spread out flat.

### DESCRIPTION OF EXEMPLARY EMBODIMENTS

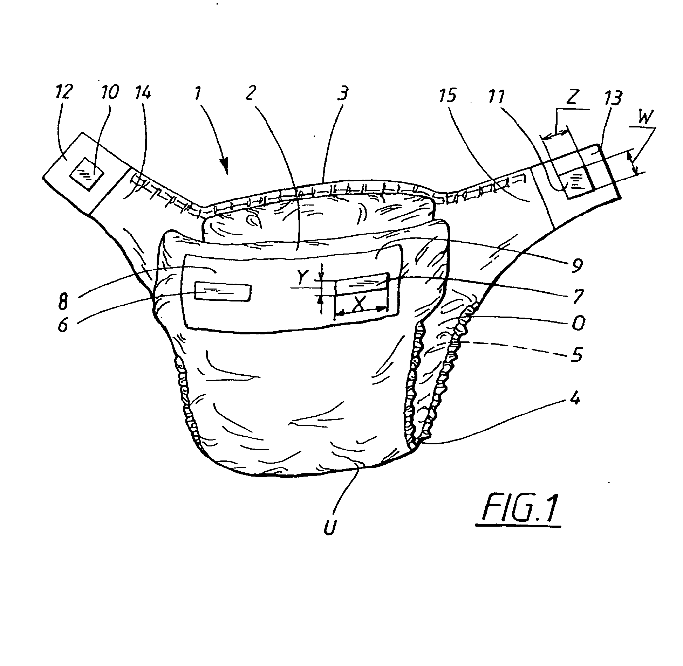

The product 1 shown in Figure 1 is an incontinence protection, i.e. an absorbent product having a longitudinal direction, a transverse direction, an upper side O, which consists of a liquid-permeable surface layer, and a lower side U which consists of a liquid-impermeable surface layer. In addition, the product 1 includes a front waist section 2, a back waist section 3, a middle section 4, which is located between the waist sections 2, 3, and an absorbent body 5 (not visible in Figure 1), which front waist section 2 is provided with front fastening members 6, 7 and has first regions 8, 9 in connection with the said front fastening members 6, 7, and which back waist section 3 is provided with back fastening members 10, 11 and has second regions 12, 13 in connection with the said back fastening members 10, 11. In the product 1 which is described, the back fastening members 10, 11 and the associated second regions 12, 13 are arranged on projecting tongues 14, 15, which projecting tongues 14, 15 extend substantially in the transverse direction of the product.

The longitudinal direction of the product extends from the back waist section 3 to the front waist section 2, and the transverse direction of the product goes transversely with respect to the said longitudinal direction, with the back waist section 3 being the waist section which bears against the seat or back of a user and the front waist section 2 being the waist section which bears against the abdomen of a user, when the product 1 is being used.

The upper side O consists of a liquid-permeable surface layer which can, for example, be a perforated plastic film, a plastic net or a textile material, a non-woven material, or a laminate of two or more material layers of this nature. The plastic materials which are employed in liquid-permeable surface materials are usually thermoplastic materials such as polyethene or polypropene. The term non-woven material refers to non-woven fabrics. Suitable non-woven materials can consist of natural fibres, such as cellulose or cotton, or of synthetic fibres, such as polyethene, polypropene, polyester, polyurethane, nylon or regenerated cellulose. It is also possible to use non-woven materials which have been produced from fibres which include two or more components, and also mixtures of different fibre types.

The liquid-permeable surface layer in the upper side O is intended to receive liquid and conduct it into the absorption body 5. Furthermore, the said surface layer should be able to prevent so-called rewetting, that is prevent absorbed body liquid from penetrating back out of the absorption body 5.

The lower side U consists of a liquid-impermeable surface layer which can be composed of thin, liquid-impermeable plastic films. However, it is also possible to use, for the liquid-impermeable surface layer, materials which were originally liquid-permeable but which have been provided with a coating of plastic, resin or other liquid-impermeable material. This prevents liquid leaking from the lower side U of the product 1. The liquid-impermeable surface layer in the lower side U can consequently consist of any optional material which is expediently kind to the skin and which fulfils the criterion of being liquid-impermeable. Examples of materials which are suitable for use as barrier layers are plastic films, non-woven materials, for example laminated non-woven materials, and also different types of laminate. Examples of plastic films which can be used are those which consist of polyethene, polypropene or polyester.

Alternatively, the liquid-impermeable surface layer in the lower side U can consist of a laminate which is composed of a liquid-permeable plastic layer which faces the absorption body and a non-woven layer which faces the underclothes of the user. Suitable non-woven materials can consist of natural fibres, such as cellulose or cotton, or of synthetic fibres, such as polyethene, polypropene, polyester, polyurethane, nylon or regenerated cellulose. It is also possible to use non-woven materials which have been produced from fibres which include two or more components and also from mixtures of different fibre types. Such a construction provides a leakage-safe barrier layer having a textile feel and also makes possible reception, and a certain co-operation, between the non-woven material, which should then include the regions 8, 9, and also 12, 13, and the fastening members 10, 11, and also 6, 7.

The absorption body 5 can advantageously be substantially made up of cellulose fluff pulp. The cellulose fluff pulp can be present in the form of coils, bundles or sheets which are dry-defibred and converted, in fluffed form, into a pulp mat with or without the admixture of super-absorbent materials, i.e. polymers having the ability to absorb several times their own weight of a fluid. Other utilisable natural materials can be cotton fibres, peat or similar. Utilisable synthetic materials can be absorbent or non-absorbent synthetic fibres. Furthermore, the absorbent body can also contain other components such as shape-stabilising members, liquid-spreading members, binding agents such as thermoplastic fibres, or absorbent foam materials. The absorption body 5 can also consist of optional and suitable mixtures, which are known to a person skilled in the art, of the materials and components which are enumerated here. Furthermore, the absorption body 5 can consist of a continuous layer or be made up of several different layers or parts. The absorption body 5 can, in addition, be profiled, i.e. be of different thicknesses in different parts of the product 1.

The layers which constitute the upper side O and the lower side U are connected to each other outside the absorption body 5 and around the whole of the periphery of the product 1. The said layers can be joined together in any optional and suitable manner, for example by means of gluing, sewing or welding using heat or ultrasound.

The division of the product 1 into two waist sections 2, 3 and a middle section 4 is not to be understood as meaning that there are boundaries between the different sections 2-4, but is first and foremost intended to facilitate description of the product 1, using as the point of departure the differences which exist between the different sections 2-4 depending on how they are intended to be located in relation to a user's body. Consequently, the transition between the different sections 2-4 does not take place at definite transverse lines but rather within diffuse transitional regions.

The front and back fastening members 6, 7, 10, 11 of the product can, for example, be of the type comprising re-sealable tapes having appurtenant reception zones (TLZ) consisting of reinforcing material, combinations of hook and loop members (for example Velcro®), hybrid variants, for example STEMWEB® from 3M, fastening members which are described in GB, A, 2303821, or combinations of adhesive fastening members and hook/loop members, or be of another suitable type which is known to a person skilled in the art.

The front fastening members shown in Figure 1, i.e. 6 and 7, have a first extent, X, which is substantially in the transverse direction of the product, and a second extent, Y, which is substantially in the longitudinal direction of the product. Furthermore, the back fastening members, 10 and 11, also shown in Figure 1, have a third extent, Z, which is substantially in the transverse direction of the product, and a fourth extent, W, which is substantially in the longitudinal direction of the product.

Furthermore, the said first regions 8, 9, in connection with the said front fastening members 6, 7, and/or the said second regions 12, 13, in connection with the said back fastening members 10, 11, consist of a material which has a character, for example surface structure, adhesion ability or the like, for example non-woven materials, hot-melt-type adhesives, fibre cloths, textile materials, loop and, hook materials or films, which make possible reception of, and a certain co-operation with, the fastening members to which the regions are not connected.

When said front and back fastening members 6, 7, 10, 11 co-operate, a fastening ability, F1, is exhibited, which fastening ability consists of the said first shearing strength, S1, and the said first peeling strength, P1. The said shearing strength, S1, can be measured by the previously mentioned method in the patent US, A, 4699622, Toussant et al., and the said peeling strength, P1, can be measured by the previously mentioned method in the patent US, A, 4846815, Scripps.

The said second shearing strength, S2, and the said second peeling strength, P2, both constituting the said fastening ability, F2, which is exhibited when the said first regions, 8 and 9, co-operate with the said back fastening members, 10 and 11, and also the said third shearing strength, S3, and the said third peeling strength, P3, both constituting the said third fastening ability, F3, which is exhibited when the said second regions, 12 and 13, co-operate with the said front fastening members, 6 and 7, can also be measured using the above mentioned methods.

By using the said methods for measuring shearing strength and peeling strength, i.e. fastening ability, it has been found that a product, 1, in accordance with the present invention exhibits a first shearing strength ratio, S2/S1, between the said second shearing strength, S2, and the said first shearing strength, S1, and also a second shearing strength ratio, S3/S1, between the said third shearing strength, S3, and the said first shearing strength, S1, both of which shearing strength ratios, S2/S1 and S3/S1, are less than 1.0, and a first peeling strength ratio, P2/P1, between the said second peeling strength, P2, and the said first peeling strength, P1, and also a second peeling strength ratio, P3/P1, between the said third peeling strength, P3, and the said first peeling strength, P1, both of which peeling strength ratios, P2/P1 and P3/P1, are less than 10, with at least one of the said second and third shearing strengths, S2 and S3, being greater than zero and/or at least one of the said second and third peeling strengths, P2 and P3, being greater than zero.

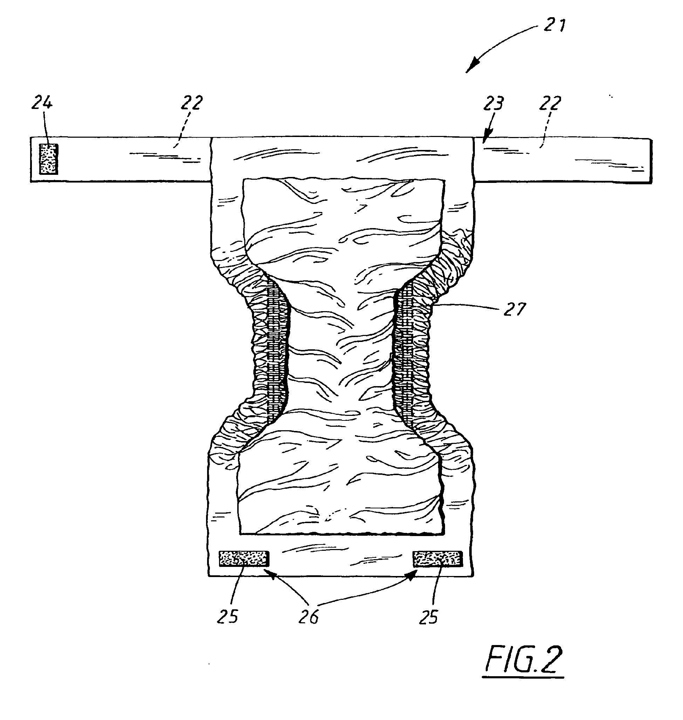

The product 21 shown in Figure 2 describes yet another type of product according to the present invention, which product is an absorbent garment in which back fastening members 22 (not shown in Figure 2) are located on a belt 23 which is integrated with the absorbent garment. The integrated belt 23 has a fastening device 24 at one end of the belt, which fastening device 24 is for fastening the belt 23 to itself. In addition, the product 21 includes front fastening members 25, first regions 26 in connection with the said front fastening members 25, and absorbent material 27. The product 21 also includes back fastening members 22 (not shown in Figure 2) and second regions (not shown in Figure 2) in connection with the said back fastening members 22. When the belt 23 is fastened to itself by the fastening device 24 being fastened to the belt 23, the said front fastening members 25 can in turn be fastened to the belt 23, with the product 21 being fastened on a user. For a more detailed description of a product according to the present invention it is referred to the description of product 1 in accordance with Figure 1.

## Claims

1. Product (1), which product (1) is absorbent or can act as a support for an absorbent product, with the product (1) having a longitudinal direction, a transverse direction, an upper side (O) and a lower side (U), and the product (1) comprises a front waist section (2), a back waist section (3) and a middle section (4) which is located between the waist sections (2, 3), which front waist section (2) is provided with front fastening members (6, 7) and has first regions (8, 9) in connection with the said front fastening members (6, 7), which back waist section (3) is provided with back fastening members (10, 11) and has second regions (12, 13) in connection with the said back fastening members (10, 11), with the said front or back fastening members (6, 7, 10, 11 ) being able to receive and co-operate with the other of the said front or back fastening members (6, 7, 10, 11), respectively, and a first fastening ability (F1) thereby being exhibited between the said front and back fastening members (6, 7, 10, 11), and the said first fastening ability (F1) comprises a first shearing strength (S1) and a first peeling strength (P1), and the said front fastening members (6, 7) or the said back fastening members (10, 11) have a first extent (Y) in the longitudinal direction of the product, and the other of the said front fastening members (6, 7) or said back fastening members (10, 11), respectively, have a second extent (W) in the longitudinal direction of the product, **characterized in that** the said first regions (8, 9) constitute the whole of the said upper side (O) or the whole of the said lower side (U), and/or **in that** the said second regions (12, 13) constitute the other of the whole of the said upper side (O) or the whole of the said lower side (U), respectively and **in that** an extent ratio (W/Y) is exhibited between the said second extent (W) and the said first extent (Y), with the said extent ratio (W/Y) being at least 1.5, and **in that** the said first regions (8, 9) can receive and co-operate with the said back fastening members (10, 11), and a second fastening ability (F2) is thereby exhibited between the said first regions (8, 9) and the said back fastening members (10, 11), and the said second fastening ability (F2) comprises a second shearing strength (S2) and a second peeling strength (P2), and/or **in that** the said second regions (12, 13) can receive and co-operate with the said front fastening members (6, 7), and a third fastening ability (F3) is thereby exhibited between the said secondary regions (12, 13) and the said front fastening members (6, 7), and the said third fastening ability (F3) comprises a third shearing strength (S3) and a third peeling strength (P3), with a first shearing strength ratio (S2/S1) being exhibited between the said second shearing strength (S2) and the said first shearing strength (S1), and a second shearing strength ratio (S3/S1) being exhibited between the said third shearing strength (S3) and the said first shearing strength (S1), both of which shearing strength ratios (S2/S1, S3/S1) are less than 1.0, and a first peeling strength ratio (P2/P1) is exhibited between the said second peeling strength (P2) and the said first peeling strength (P1), and a second peeling strength ratio (P3/P1) is exhibited between the said third peeling strength (P3) and the said first peeling strength (P1), both of which peeling strength ratios (P2/P1, P3/P1) are less than 10, with at least one of the said second and third shearing strengths (S2, S3) being greater than zero and/or at least one of the said second and third peeling strengths (P2, P3) being greater than zero.

2. Product (1) according to claim 1, **characterized in that** the said shearing strength ratios (S2/S1, S3/S1) are less than 0.8, and/or the said peeling strength ratios (P2/P1, P3/P1) are less than 7.

3. Product (1) according to claim 1 or 2, **characterized in that** the said first shearing strength ratios (S2/S1) and/or the said second shearing strength ratios (S3/S1) are greater than 0.01, and/or the said first peeling strength ratios (P2/P1) and/or the said second peeling strength ratios (P3/P1) are greater than 0.05.

4. Product (1) according to any one of the preceding claims,
**characterized in that** the said first regions (8, 9) constitute an area of the said front waist section (2) or are restricted to a relatively small area, or **in that** the said second regions (12, 13) constitute a relatively large area of the said back waist section (3) or are substantially restricted to a relatively small area.

5. Product (1) according to any one of the preceding claims, **characterized in that** the said front and/or back fastening members (6, 7, 10, 11) is/are arranged on projecting tongues (14, 15), which projecting tongues (14. 15) are arranged at the said front and/or back waist section (2, 3).

6. Product (1) according to any one of the preceding claims, **characterized in that** the said first regions (8, 9) and/or the said second regions (12, 13) consist of non-woven material.

7. Product (1) according to any one of the preceding claims, **characterized in that** the said lower side (U) consists of non-woven material, which non-woven material is laminated to form a liquid-impermeable layer.

8. Product (1) according to any one of the preceding claims, **characterized in that** the said fastening members (6, 7, 10, 11) consist of co-operating hook and loop elements.

9. Product (1) according to any one of the preceding claims, **characterized in that** the said fastening members (6, 7, 10, 11) consist of co-operating re-sealable tapes having appurtenant reception zones consisting of reinforcing material.

10. Product (1) according to any one of the preceding claims, **characterized in that** the said product (1) is an absorbent product, which absorbent product (1) includes an absorbent body (5).

11. Product (1) according to any one of the preceding claims, **characterized in that** said product (1) is a non-absorbent product.

## Patentansprüche

1. Produkt (1), das absorbierend ist oder als ein Träger für ein absorbierendes Produkt dienen kann, wobei das Produkt (1) eine Längsrichtung, eine Querrichtung, eine Oberseite (O) und eine Unterseite (U) aufweist, und einen vorderen Taillenabschnitt (2), einen hinteren Taillenabschnitt (3) und einen Mittelabschnitt (4), der zwischen den Taillenabschnitten (2, 3) angeordnet ist, umfasst, wobei der vordere Taillenabschnitt (2) mit vorderen Verschlusselementen (6, 7) versehen ist und erste Bereiche (8, 9) in Verbindung mit den vorderen Verschlusselementen (6, 7) aufweist, der hintere Taillenabschnitt (3) mit hinteren Verschlusselementen (10, 11) versehen ist und zweite Bereiche (12, 13) in Verbindung mit den hinteren Verschlusselementen (10, 11) aufweist, wobei die vorderen oder hinteren Verschlusselemente (6, 7, 10, 11) in der Lage sind, die anderen der vorderen oder hinteren Verschlusselemente (6, 7, 10, 11) entsprechend aufzunehmen und mit diesen zusammenzuwirken, wodurch eine erste Verschlussfähigkeit (F1) zwischen den vorderen und hinteren Verschlusselementen (6, 7, 10, 11) bewirkt wird, die eine erste Scherkraft (S1) und eine erste Ablösekraft (P1) umfasst, und wobei die vorderen Verschlusselemente (6, 7) oder die hinteren Verschlusselemente (10, 11) eine erste Dimension (Y) in Längsrichtung des Produkts aufweisen und die anderen der vorderen Verschlusselemente (6, 7) oder der hinteren Verschlusselemente (10, 11) entsprechend eine zweite Dimension (W) in der Längsrichtung des Produkts aufweisen, **dadurch gekennzeichnet, dass** die ersten Bereiche (8, 9) die gesamte Oberseite (O) oder die gesamte Unterseite (U) bilden und/oder **dadurch**, dass die zweiten Bereiche (12, 13) entsprechend die andere der gesamten Oberseite (O) oder der gesamten Unterseite (U) bilden, und **dadurch**, dass ein Dimensionsverhältnis (W/Y) zwischen der zweiten Dimension (W) und der ersten Dimension (Y) vorliegt, wobei das Dimensionsverhältnis (W/Y) wenigstens 1,5 beträgt, und **dadurch**, dass die ersten Bereiche (8, 9) die hinteren Verschlusselemente (10, 11) aufnehmen und mit diesen zusammenwirken können, und **dadurch** eine zweite Verschlussfähigkeit (F2) zwischen den ersten Bereichen (8, 9) und den hinteren Verschlusselementen (10, 11) bewirkt wird, die eine zweite Scherkraft (S2) und eine zweite Ablösekraft (P2) aufweist, und/oder **dadurch**, dass die zweiten Bereiche (12, 13) die vorderen Verschlusselemente (6, 7) aufnehmen und mit diesen zusammenwirken können, wodurch eine dritte Verschlussfähigkeit (F3) zwischen den zweiten Bereichen (12, 13) und den vorderen Verschlusselementen (6, 7) bewirkt wird, die eine dritte Scherkraft (S3) und eine dritte Ablösekraft (P3) aufweist, wobei das Scherkraftverhältnis (S2/S1), das zwischen der zweiten Scherkraft (S2) und der ersten Scherkraft (S1) vorliegt, und ein zweites Scherkraftverhältnis (S3/S1), das zwischen der dritten Scherkraft (S3) und der ersten Scherkraft (S1) vorliegt, beide geringer als 1,0 sind, und ein erstes Ablösekraftverhältnis (P2/P1), das zwischen der zweiten Ablösekraft (P2) und der ersten Ablösekraft (P1) vorliegt, und ein zweites Ablösekraftverhältnis (P3/P1), das zwischen der dritten Ablösekraft (P3) und der ersten Ablösekraft (P1) vorliegt, geringer als 10 sind, wobei wenigstens die zweite oder dritte Scherkraft (S2/S3) größer als Null ist und/oder wenigstens die zweite oder dritte Ablösekraft (P2, P3) größer als Null ist.

2. Produkt (1) nach Anspruch 1, **dadurch gekennzeichnet, dass** die Scherkraftverhältnisse (S2/S1, S3/S1) geringer als 0,8 sind und/oder die Ablösekraftverhältnisse (P2/P1, P3/P1) geringer als 7 sind.

3. Produkt (1) nach Anspruch 1 oder 2, **dadurch gekennzeichnet, dass** das erste Scherkraftverhältnis (S2/S1) und/oder das zweite Scherkraftverhältnis (S3/S1) größer als 0,01 sind und/oder das erste Ablösekraftverhältnis (P2/P1) und/oder das zweite Ablösekraftverhältnis (P3/P1) größer als 0,05 sind.

4. Produkt (1) nach einem der vorstehenden Ansprüche, **dadurch gekennzeichnet, dass** die ersten Bereiche (8, 9) einen Bereich des vorderen Taillenabschnitts (2) bilden oder auf einen relativ kleinen Bereich beschränkt sind, oder **dadurch**, dass die zweiten Bereiche (12, 13) einen relativ großen Bereich des hinteren Taillenabschnitts (3) bilden oder im Wesentlichen auf einen relativ kleinen Bereich beschränkt sind.

5. Produkt (1) nach einem der vorstehenden Ansprüche, **dadurch gekennzeichnet, dass** die vorderen und/oder hinteren Verschlusselemente (6, 7, 10, 11) auf vorragenden Zungen (14, 15) angeordnet sind, wobei die vorragenden Zungen (14, 15) an dem vorderen und/oder hinteren Taillenabschnitt (2, 3) angeordnet sind.

6. Produkt (1) nach einem der vorstehenden Ansprüche, **dadurch gekennzeichnet, dass** die ersten Bereiche (8, 9) und/oder die zweiten Bereiche (12, 13) aus einem Vliesstoff bestehen.

7. Produkt (1) nach einem der vorstehenden Ansprüche, **dadurch gekennzeichnet, dass** die untere Seite (U) aus einem Vliesstoff besteht, welcher vliesstoffbeschichtet ist, um eine flüssigkeitsundurchlässige Lage zu bilden.

8. Produkt (1) nach einem der vorstehenden Ansprüche, **dadurch gekennzeichnet, dass** die Verschlusselemente (6, 7, 10, 11) aus zusammenwirkenden Klettverschlusselementen bestehen.

9. Produkt (1) nach einem der vorstehenden Ansprüche, **dadurch gekennzeichnet, dass** die Verschlusselemente (6, 7, 10, 11) aus zusammenwirkenden wiederverschließbaren Klebestreifen bestehen, die' zugehörige Aufnahmebereiche aufweisen, die aus Verstärkungsmaterial bestehen.

10. Produkt (1) nach einem der vorstehenden Ansprüche, **dadurch gekennzeichnet, dass** das Produkt (1) ein absorbierendes Produkt ist, das einen Absorptionskörper (5) umfasst.

11. Produkt (1) nach einem der vorstehenden Ansprüche, **dadurch gekennzeichnet, dass** das Produkt (1) ein nichtabsorbierendes Produkt ist.

## Revendications

1. Article (1), lequel article (1) est absorbant ou peut servir de support pour un produit absorbant, l'article (1) ayant une direction longitudinale, une direction transversale, une face supérieure (O) et une face inférieure (U), et l'article (1) comprend une section taille avant (2), une section taille arrière (3) et une section médiane (4) qui est située entre les sections de taille (2, 3), laquelle section taille avant (2) est pourvue d'éléments de fermeture avant (6, 7) et a des premières régions (8, 9) en connexion avec lesdits éléments de fermeture avant (6, 7), laquelle section taille arrière (3) est pourvue d'éléments de fermeture arrière (10, 11) et a des deuxièmes régions (12, 13) en connexion avec lesdits éléments de fermeture arrière (10, 11), lesdits éléments de fermeture avant ou arrière (6, 7, 10, 11) étant aptes à recevoir et à coopérer avec l'autre desdits éléments de fermeture avant ou arrière (6, 7, 10, 11), respectivement, et une première capacité de fermeture (F1) étant de ce fait présente entre lesdits éléments de fermeture avant et arrière (6, 7, 10, 11), et ladite première capacité de fermeture (F1) comprend une première résistance au cisaillement (S1) et une première résistance au pelage (P1), et lesdits éléments de fermeture avant (6, 7) ou lesdits éléments de fermeture arrière (10, 11) ont une première dimension (Y) dans la direction longitudinale de l'article, et les autres desdits éléments de fermeture avant (6, 7) ou desdits éléments de fermeture arrière (10, 11), respectivement, ont une deuxième dimension (W) dans la direction longitudinale de l'article, **caractérisé en ce que** lesdites premières régions (8, 9) constituent l'ensemble de ladite face supérieure (O) ou l'ensemble de ladite face inférieure (U), et/ou **en ce que** lesdites deuxièmes régions (12, 13) constituent l'autre de l'ensemble de ladite face supérieure (O) ou de l'ensemble de ladite face inférieure (U), respectivement, et **en ce qu'**il y a un rapport de dimensions (W/Y) entre ladite deuxième dimension (W) et ladite première dimension (Y), ledit rapport de dimensions (W/Y) étant supérieur ou égal à 1,5, et **en ce que** lesdites premières régions (8, 9) peuvent recevoir et coopérer avec lesdits éléments de fermeture arrière (10, 11), et une deuxième capacité de fermeture (F2) comprend une deuxième résistance au cisaillement (S2) et une deuxième résistance au pelage (P2), et/ou **en ce que** lesdites deuxièmes régions (12, 13) peuvent recevoir et coopérer avec lesdits éléments de fermeture avant (6, 7), et une troisième capacité de fermeture (F3) existe de ce fait entre lesdites deuxièmes régions (12, 13) et lesdits éléments de fermeture avant (6, 7), et ladite troisième capacité de fermeture (F3) comprend une troisième résistance au cisaillement (S3) et une troisième résistance au pelage (P3), un premier rapport de résistances au cisaillement (S2/S1) existant entre ladite deuxième résistance au cisaillement (S2) et ladite première résistance au cisaillement (S1), et un deuxième rapport de résistances au cisaillement (S3/S1) existant entre ladite troisième résistance au cisaillement (S3) et ladite première résistance au cisaillement (S1), lesdits rapports de résistances au cisaillement (S2/S1, S3/S1) étant tous deux inférieurs à 1,0, et un premier rapport de résistances au pelage (P2/P1) existant entre ladite deuxième résistance au pelage (P2) et ladite première résistance au pelage (P1), et un deuxième rapport de résistances au pelage (P3/P1) existant entre ladite troisième résistance au pelage (P2) et ladite première résistance au pelage (P1), lesdits rapports de résistances au pelage (P2/P1, P3/P1) étant tous deux inférieurs à 10, avec au moins l'une desdites deuxième et troisième résistances au cisaillement (S2, S3), supérieure à zéro et/ou au moins l'une desdites deuxième et troisième résistances au pelage (P2, P3) supérieure à zéro.

2. Article (1) selon la revendication 1, **caractérisé en ce que** lesdits rapports de résistances au cisaillement (S2/S1, S3/S1) sont inférieurs à 0,8, et/ou lesdits rapports de résistances au pelage (P2/P1, P3/P1) sont inférieurs à 7.

3. Article (1) selon la revendication 1 ou 2, **caractérisé en ce que** lesdits premiers rapports de résistances au cisaillement (S2/S1) et/ou lesdits deuxièmes rapports de résistances au cisaillement (S3/S1) sont supérieurs à 0,01, et/ou lesdits premiers rapports de résistances au pelage (P2/P1) et/ou lesdits deuxièmes rapports de résistances au cisaillement (P3/P1) sont supérieurs à 0,05.

4. Article (1) selon l'une quelconque des revendications précédentes, **caractérisé en ce que** lesdites premières régions (8, 9) constituent une zone de ladite section taille avant (2) ou bien sont limitées à une zone relativement petite, ou **en ce que** lesdites deuxièmes régions (12, 13) constituent une zone relativement grande de ladite section taille arrière (3) ou bien sont substantiellement limitées à une zone relativement petite.

5. Article (1) selon l'une quelconque des revendications précédentes, **caractérisé en ce que** lesdits éléments de fermeture avant et/ou arrière (6, 7, 10, 11) sont placés sur des languettes saillantes (14, 15), lesquelles sont placées sur ladite section taille avant et/ou arrière (2, 3).

6. Article (1) selon l'une quelconque des revendications précédentes, **caractérisé en ce que** lesdites premières régions (8, 9) et/ou lesdites deuxièmes régions (12, 13) sont constituées de matériau non tissé.

7. Article (1) selon l'une quelconque des revendications précédentes, **caractérisé en ce que** ladite face inférieure (U) est constituée de matériau non tissé, lequel matériau est stratifié pour former une couche imperméable aux liquides.

8. Article (1) selon l'une quelconque des revendications précédentes, **caractérisé en ce que** lesdits éléments de fermeture (6, 7, 10, 11) sont constitués d'éléments à crochets et à boucles qui coopèrent.

9. Article (1) selon l'une quelconque des revendications précédentes, **caractérisé en ce que** lesdits éléments de fermeture (6, 7, 10, 11) sont constitués de rubans refermables qui coopèrent comportant des zones de réception annexes constituées de matériau de renforcement.

10. Article (1) selon l'une quelconque des revendications précédentes, **caractérisé en ce que** ledit article (1) est un article absorbant, lequel comprend un corps absorbant (5).

11. Article (1) selon l'une quelconque des revendications précédentes, **caractérisé en ce que** ledit article (1) est un article non absorbant.
